# EUROPEAN PATENT SPECIFICATION

(11) **EP 1 170 216 B1**
(45) Date of publication and mention of the grant of the patent: **17.05.2006**
(21) Application number: 01115623.9
(22) Date of filing: 03.07.2001
(51) Int. Cl.: B65B 43/44, B65G 59/10

(54) **Device for picking up tubes from a box and placing them onto a conveyor**
Gerät zum Ergreiffen von Tuben aus einer Schachtel und zur Ablage auf ein Förderband
Dispositif pour saisir des tubes dans une boIte et les déposer sur un convoyeur

(30) Priority: 06.07.2000 IT BO000407
(43) Date of publication of application: 09.01.2002
(73) Proprietor: Tonazzi S.r.l., 20023 Cerro Maggiore (Milano) (IT)
(72) Inventor: Marchesini, Maurizio, 40065 Pianoro (Bologna) (IT)
(74) Representative: Dall'Olio, Giancarlo

(56) References cited:
- DE-A- 3 631 891
- US-A- 5 524 416

## Description

The present invention relates to automatic and half-automatic units for filling and sealing tube containers.

More in detail, the present invention relates to an operative unit for picking up tubes from a box-like package and placing the picked up tubes onto at least one conveying line.

The proposed operative unit can be used e.g. between a line for feeding box-like packages containing tubes to be filled, coming from a collecting unit, and at least one line for conveying tubes to tubes filling and closing machine.

Generally, according to known and widely used techniques, the just formed tubes have a cylindrical or truncated cone shape and their end opposite to the one with safety and guarantee cap, i.e. the end forming the mouth for introducing the filling product or substance, is open.

The so formed tubes are arranged one beside another to form a group inside suitable box-like packages, whose top wall is open.

The tubes are introduced into the packages and arranged parallel one to another and to the lateral walls of the packages, so that the ends with the caps are turned toward the bottom of the box-like package and the mouth for introducing the product is turned toward the open top wall of the packages.

The so-filled box-like packages are arranged on conveying lines with the open top wall turned upwards, so as to be conveyed to the tubes filling and closing machine.

It is possible that, during transportation to this machine, impurities and/or atmospheric dust present in the environment surrounding the production equipment, deposit and penetrate into the tubes through the mouth turned upwards.

The introduction and depositing of the atmospheric impurities inside the tubes is burdensome and harmful, because the inside of the tubes is no longer aseptic.

Another disadvantage of the transport of the tubes from the box-like packages to the conveying line, which carries the tubes to the tubes filling and closing machine results from the operating techniques of the tubes picking up.

Actually, the unit for picking up the tubes and putting them onto the conveying line is equipped with operative heads formed by extractor arms, which can be opened wide.

The extractor arms are introduced into tubes through the relative mouths and then, they are opened to abut against and grip the tubes inner walls.

However, the introduction of the extractor arms into the tubes provokes rubbing against the walls and consequently, the detachment of the sealing adhesive present on the tubes inner walls in correspondence to the mouth.

Therefore, adhesive material can be conveyed into the tube and contaminate and change of the chemical-physical characteristics of the product, which is later introduced therein.

This fact, especially in case of foodstuff or articles for personal use, can be an unpleasant and sometimes, harmful disadvantage for consumers.

The object of the present invention is to propose an operative unit for picking up tubes from a box-like package and for placing them onto a conveying line, which outweighs the above mentioned drawbacks.

Another object of the present invention is to propose an operative unit, which reduces drastically the possibility that impurities or atmospheric dust penetrate into the tubes, situated inside the box-like packages, during their transfer toward the tubes filling and closing machine.

In particular, the main object of the present invention is to propose a unit for picking up tubes from a box-like package and for placing them onto conveying lines, which operate in germ-free isolated environment.

Another main object of the present invention is to propose an operative unit, which picks up the tubes from the box-like packages without touching the tubes inner walls and without interfering with the adhesive material present in the tubes mouth.

A further object of the present invention is to propose an operative unit obtained by a simple technical solution and from extremely functional elements, which optimize the picking up of tubes from the box-like packages and placing them on the corresponding conveying lines.

A still further object of the present invention is to propose an operative unit which ensures high reliability during tubes picking up as well as during the transfer and placing onto the conveying line, thus ensuring constantly high performance of the production cycle.

The characteristic features of the present invention will be pointed out in the following description of a preferred, but not only embodiment, with reference to the enclosed drawings, in which:
- Figure 1 is a schematic lateral view of the proposed operative unit as a whole, during picking up of the tubes from the box-like packages and their transfer and placing onto a conveying line;
- Figure 2 is a schematic top view of an element of the proposed unit;
- Figure 3 is a section view taken along III-III of Figure 2;
- Figure 4 is a schematic lateral view of picking up tubes from the relative package;
- Figures 5 and 6 are schematic front views of working steps related to picking up tubes by the proposed operative unit.

With reference to enclosed drawings, the reference letter U indicates the operative unit proposed by the present invention.

The operative unit U is situated between a line (not shown) for feeding box-like packages C, containing tubes T, and at least one conveying line B aimed at conveying the tubes T, placed thereon, to the tubes filling and closing machine (not shown).

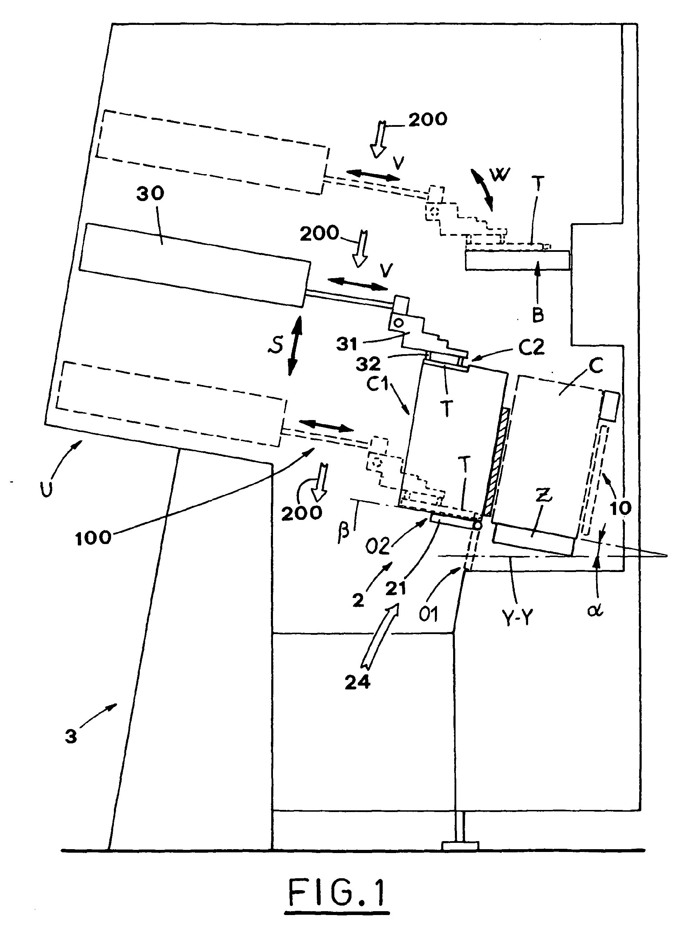

The above mentioned feeding line features a forward moving surface Z, which is inclined with respect to the horizontal plane y-y by an angle α, to define a plane β (see Figure 1).

The packages C are box-like and their top wall C1 is open, so as to allow the withdrawal of the tubes T housed therein.

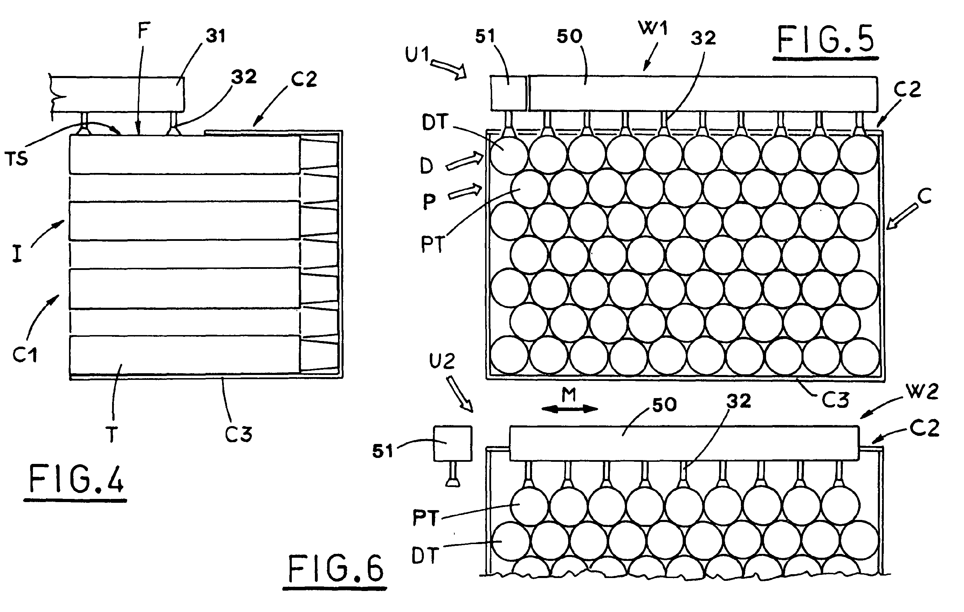

The tubes T are arranged inside the packages C parallel to each other and with their mouth I opened for introduction of the product, turned toward the open top wall C1 (Figure 4).

Moreover, one of the lateral walls C2 of the box-like packages C is equipped with a window F, adjacent to the open top wall C1, which makes the lateral walls TS of the tubes T accessible from outside and from the top (Figure 4).

The tubes T are arranged inside the packages C along subsequent odd rows D and even rows P, as shown especially in Figures 5 and 6.

The odd rows D and even rows P are situated one over another beginning from the window wall C2 up to the opposite lateral wall C3 of the package C.

Each of the odd rows D includes N+1 tubes DT, arranged one beside another, while each of the even rows P includes N tubes PT, arranged one beside another.

Each tube PT of each even row P is introduced between two corresponding tubes DT of the odd rows D and is offset with respect to the latter by e.g. half-diameter.

The wall C3 of the packages C containing the tubes T rests on the forward movement plane Z of the feeding line with the window lateral wall C2 turned upwards and with the open top wall C1 turned sideward (Figure 1).

In particular, the value of the above mentioned angle α is such as to orient the tubes T contained in the package C, in a horizontal or substantially horizontal direction.

In this way, any impurities present in the surrounding environment are prevented from entering the tubes T during the forward movement of the package C on the feeding line.

The operative unit U for picking up tubes T housed inside packages C and for transferring and placing them onto the conveying line B, is situated in correspondence to the end stop of the feeding line.

The operative unit U includes, e.g. a pusher 10, acting crosswise to the forward movement plane Z and aimed at going in abutment against the packages C to push them toward a support seat 2 of the operative unit U.

The support seat 2 includes a support plane 21, which can be rotated between two operative configurations (Figure 1): first configuration O1, in which the support plane 21 is perpendicular to the forward movement plane Z, and second configuration O2, in which the support plane 21 is aligned with the forward movement plane Z, and consequently, coplanar with the above mentioned plane β.

When in the first operative configuration O1, the support plane 21 uncovers an outlet mouth 24, so as to discharge the packages C emptied of the tubes T toward a collecting unit (not shown), while in the second operative configuration 02, the support plane supports the packages C.

Known and not shown means for stabilizing the position and consequently, orientation of the package C on the surface plane 21, are joined to the support seat 2.

According to the enclosed figures, the support plane 21, in the second operative configuration O2, and the forward movement plane Z are inclined with respect to the horizontal plane y-y by the same angle α.

It is possible to incline the support plane 21 in the configuration O2 by an angle different from the inclination angle of the forward movement plane Z.

The proposed operative unit U includes, near the support seat 2, a driving unit 30 equipped with an operative head 31.

The operative head 31 is equipped with picking up means 32 for withdrawing tubes T from the packages C.

In this connection, the packages C, arranged on the forward movement plane Z with their wall C2 turned upwards, turn the open top wall C1 toward the driving unit 30, so that, after having been transferred to the support seat 2, the operative head 31 can act directly in correspondence to the open top wall C1 as well to the window F of the lateral wall C2.

On one side, the space 100 is delimited, at the bottom, by the support and activation means 3 (not shown in detail, as not inherent to the invention) of the driving unit 30, and, -at the top, by the driving unit 30. On the other side, the space 100 is delimited, at the bottom, by the support seat 2 and, at the top, by the conveying line B.

The space 100 is clear of any operative means or elements and is occupied only by the above mentioned operative head 31.

Thus, it is possible to insert the space 100 in an isolated germ-free system and consequently, to send and convey, by known and not shown means, a vertical downward laminar flow of air 200: in this way, a "sterile area" is defined.

The laminar flow 200 extends along the conveying line B, the operative head 31 in different operative configurations of picking up and removing tubes T from the package C and placing them onto the conveying line B, and through the package C situated on the support seat 2.

Thus, the surrounding environment is constantly maintained in condition very close to aseptic.

The operative head 31, according to known techniques, is moved in a direction V parallel to the plane β to be placed in correspondence to the packages C situated on the support seat 2 in order to remove tubes, and subsequently, returns toward the driving unit 30.

Due to the raising, according to known techniques, of the driving unit 30 in a direction S practically vertical and perpendicular to the plane β, the operative head 31 can be positioned in correspondence to the conveying line B, to put down the tubes, and subsequently can return toward the driving unit 30.

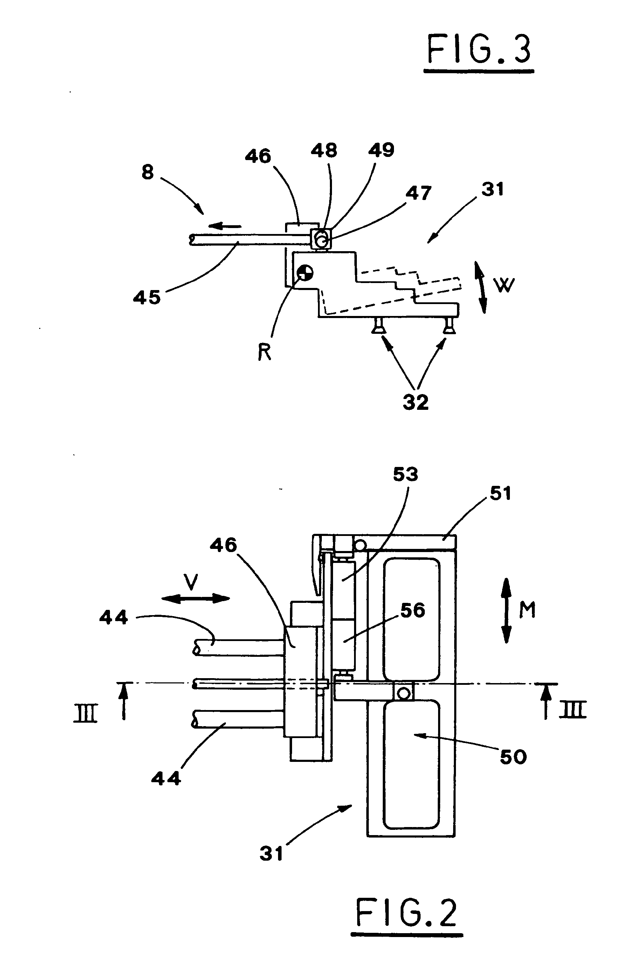

The driving unit 30 includes, situated thereinside, control means 8 which operate the operative head 31 to rotate in directions W (Figures 1 and 3).

During the positioning of the operative head 31 in correspondence to the conveying line B, the rotation control means 8 allow to determine the correct positioning of the tubes T thereon.

For this purpose, the operative head 31 is rotatably hinged to a plate 46 along a rotation axis R (Figures 2 and 3).

The operative head is operated to back-and-forth motion along the direction V by known techniques, e.g. bars 44 integral with the plate 46.

The rotation control means 8 of the operative head 31 are equipped with a bar 45, which features, at the head end, a pin 47 introduced into a slot 48 made in the body 49 of the operative head 31.

The bar 45 is connected to an actuator (not shown), which pushes the bar 45, and thus forces it, by a pin 47, to act in correspondence to the slot 48, to determine the operative head 31 rotation.

The operative head 31 is equipped with a central arm 50, which has N picking up elements 32 and with a lateral arm 51 with only one picking up element.

The picking up elements 32 can be e.g. a pair of suction cups (Figure 3).

The central arm 50 is connected to a first actuator 56, situated on the operative head 31 and aimed at moving the central arm 50 in a direction M, perpendicular to the direction V (Figure 2), between two separate operative configurations: first W1 (Figure 5) and second W2 (Figure 6), which will be defined later.

A second actuator is situated in the operative head 31 to operate the lateral arm 51 to move between two separate operative configurations (Figure 5 and 6): first, union configuration U1, in which the lateral arm 51 is contiguous to the central arm 50, and second, separated configuration U2, in which the lateral arm 51 is moved far from the central arm 50.

The operative head 31 is situated in correspondence to the package C situated in the support seat 2 and consequently, the central arm 50 and the lateral arm 51, the latter being in the first union configuration U1, are aimed at picking up N+1 tubes DT from odd rows D by acting through the window F of the wall C2 by means of the relative N+1 picking up means.

It is to be pointed out that, during this step, the tubes are gripped from the side and from the top by the picking up means 32.

For this purpose, the central arm 50 is in the first configuration W, so as to go in abutment, with the cooperation of the lateral arm 51, against the tubes from the odd rows.

The central arm 50, with the lateral arm 51 moved away in the separated configuration U2, is to be moved by the first pusher 56 in the direction M toward the second configuration W2, so as to position the relative N picking up means 32 in correspondence to the N tubes PT of the even rows P.

Operation of the proposed operative unit is described in the following with reference, as example, to picking up of the first two rows of tubes of a box-like package C.

During transferring toward the unit U, the box-like package C, situated on the forward movement plane Z, has its lateral wall C2 with the window F turned upwards, while the open head wall C1 is turned sideward.

In this position, it is extremely improbable that impurities and/or atmospheric dust penetrate into the tubes T through the mouths I turned upward, since the tubes are in horizontal or substantially horizontal position.

Possible traces of impurities and/or atmospheric dust are limited to the tubes extreme portion, in correspondence to the relative mouths, which will not get in contact with the product, because they will be folded during the sealing.

When the package C reaches the end stop of the feeding line, the pusher 10 is operated to transfer the package C to the support plane 21, situated in the second operative position 02.

In step relation with the stabilization of the package C on the support plane 21, the operative head 31 is positioned in correspondence to the window F of the wall C2 of the package C (Figure 1).

The operative head 31, together with the lateral arm 51, contiguous to the central arm 50 (configuration U1 of Figure 5) can go in abutment, from the top through the window F, and pick up, by N+1 picking up means 32, the first row (odd D) of tubes DT in correspondence to the outer lateral walls TS.

In this step, the central arm 50 is in the first configuration W1.

Thus, it is obvious that the picking up means 32 do not interfere with the inner walls of the tubes T.

In step relation, the operative head 31 returns toward the driving unit 30, thus removing the tubes DT, gripped from the top and from the side by the picking up means 32, from the package C through the open top wall C1.

Consequently, the driving unit 30 is translated in direction S and positioned at the level of the conveying line B and, in suitable step relation, the operative head 31 is translated in direction V and positioned near the conveying line B.

At this point, the rotation control means 8 of the operative head 31 are operated to make the operative head 31 rotate slightly in directions W, thus allowing the correct positioning of the tubes DT on the conveying line B.

Consequently, the picking up can release the tubes.

Then, the operative head 31 returns toward the driving unit 30, which is lowered again to the level of the window wall C2 of the package C.

The second actuator 53 of the operative head 31 separates the lateral arm 51 from the central arm 50 (configuration U2), and consequently, the operative head 31 is moved toward the package C.

The first actuator 56 moves the central arm 50 crosswise in the direction M toward the second configuration W2, so as to position the N picking up means 32 near the tubes PT (N tubes) of the second row of tubes in the package (even row P) (Figure 6).

Thus, the tubes of the second row of the package C are reached and gripped, at their outer lateral walls TS, from the top through the window F.

The whole above described operative cycle containing return of the operative head 31, raising of the driving unit 30, positioning of the operative head 31 on the conveying line B, placing the tubes, return of the operative head 31, lowering of the driving unit 30 toward the package C, is repeated.

Thus, the proposed operative unit manages to remove the tubes from the package and to transfer and place them onto the conveying line B by simple and immediate operations, without acting on the tubes inner walls.

Moreover, during the above described operative steps, the laminar flow 200 allows to maintain the surrounding environment practically aseptic, preventing the impurities and atmospheric dust from depositing and penetrating into the tubes.

In step relation with the complete emptying of the package C, the support plane 21 returns to the first operative configuration O1 (Figure 1), thus opening the outlet mouth 24, so as to discharge the emptied package to the collecting unit.

From what above, it is evident how the proposed operative unit manages, in an extremely advantageous way, to drastically reduce the possibility that the tubes can be contaminated by impurities and/or atmospheric dust.

The particular inclination, identical or slightly different, of the line for feeding packages C and of the support plane 21 in configuration O2 ensures, from one side, that the tubes will be oriented substantially horizontal, and from the other side, that during different operative steps, the tubes will be kept in their predetermined position inside the packages.

Moreover, the proposed operative unit advantageously allows to remove the tubes from the packages without interfering with the tubes inner walls.

It is also to be pointed out that the space 100 is occupied only by the operative head 31.

It is also to be noted that the laminar flow 200, conveyed downwards in the space 100, allows to maintain the environment practically aseptic.

It is still to be noted that the proposed operative unit is obtained by an extremely simple, but highly functional technical solution, which optimizes the picking up of the tubes from the packages as well as the transferring and placing them onto the conveying lines.

Another advantage of the proposed operative unit derives from the fact that it is obtained by few elements, which are simple in relation to their performance, and consequently, the production costs are very low.

Consequently, during their transferring from the package C to the conveying line B, the tubes are kept practically horizontal, and are gripped from the top and in correspondence to their outer walls, passing through the sterile area 100.

## Claims

1. Operative unit for picking up tubes from a box-like package and placing them onto a conveying line, said operative unit (U) being situated between at least one line for feeding said box-like packages (C) and at least one conveying line (B), said box-like packages (C) having a top wall (C1) open for picking up said tubes (T), which are arranged inside the packages (C) with the mouth (I) for introducing the product turned toward the open top wall (C1), **characterized in that** it includes: a support seat (2) which receives and stabilizes a relative package (C) of said box-like packages, coming from said feeding line, said package (C) having the upper lateral wall (C2) provided with a window (F) adjacent to said open top wall (C1); a driving unit (30), situated near said support seat (2) and equipped with an operative head (31) with picking up means (32), said driving unit (30) being aimed at operating said operative head (31) to convey the latter to said window (F) of said package (C) situated in said support seat (2), so as to turn the open top wall (C1) toward the driving unit (30) and to operate said picking up means (32) to grip the outer lateral wall (TS) of said tubes (T) from the top, through said window (F), said driving unit (30) being also aimed at making said operative head (31) return, in step relation, to remove said tubes (T) from said package (C) through the open top wall (C1), and be brought to said conveying line (B) for placing said tubes (T) thereon.

2. Unit, according to claim 1, **characterized in that** a space (100) which is delimited, at one side, by said driving unit (30), and, at the other side, by the support seat (2) at the bottom and by the conveying line (B) at the top, is occupied only by said operative head (31).

3. Unit, according to claim 2, **characterized by** means for sending and conveying, vertically downwards through said space (100), a laminar air flow (200), which extends through said conveying line (B), said operative head (31) in different operative configurations of picking up and removing tubes (T) from the package (C) and placing them onto the conveying line (B), and through said package (C) situated on said support seat (2).

4. Unit, according to claim 1, **characterized in that** the forward moving surface (Z) of said feeding line forms a plane (β) inclined with respect to a horizontal plane (y-y) by an angle (α), with the opening of said angle (α) turned toward the driving unit (30) and **in that** said support seat (29 includes a support plane (21), which can be rotated between two different operative configurations: first rest configuration (O1), in which said support plane (21) is perpendicular to said plane (β) and second operative configuration (O2), in which said support plane (21) is aligned with said plane (β) to support a package (C).

5. Unit, according to claim 4, **characterized in that** the value of said angle (α) is such that the tubes (T) contained in said package (C) are oriented in a horizontal or substantially horizontal direction during the forward movement of the package (C) on said feeding line and transfer to said support seat (2).

6. Unit, according to claim 4, **characterized in that** said support plane (21) in said first rest configuration (O1) uncovers an outlet mouth (24), so as to discharge the packages (C) emptied of the tubes (T) toward a collecting unit.

7. Unit, according to claims 1 and 4, **characterized in that** it includes supporting and operating means (3) of said driving unit (30) which moves the latter in a direction (S), perpendicular to said plane (β), from said support seat (2) to said conveying line (B), and vice-versa.

8. Unit, according to claims 1 and 4, **characterized in that** said driving unit (30) operates said operative head (31) to alternate motion in a direction (V), parallel to said plane (β).

9. Unit, according to claim 1, **characterized in that** it includes, situated inside said driving unit (30), control means (8) which operate the operative head (31) to rotate in directions (W), during the positioning of said operative head (31) in correspondence to said conveying line (B) said control means (8) being aimed at orienting tubes (T), carried by said operative head (31), parallel thereto.

10. Unit, according to claim 9, **characterized in that** said rotation control means (8) include a bar (45), which features, at its end, a pin (47) introduced into a slot (48) made in the body (49) of the operative head (31), said bar (45) being pushed by an actuator, to operate, by the relative movement of said pin (47) within said slot (48), the operative head (31) to rotate on a rotation axis (R).

11. Unit, according to claim 1, **characterized in that** said tubes (T) are arranged in said packages (C) with the mouth (I) turned toward the open top wall (C1) and along subsequent odd rows (D) and even rows (P) said odd rows (D) and even rows (P) being situated one over another beginning from the lower lateral wall (C3) up to the upper lateral window wall (C2), with each of the odd rows (D) including N+1 tubes (DT) of said tubes (T), arranged one beside another, and with each of the even rows (P) including N tubes (PT) of said tubes (T), arranged one beside another.

12. Unit, according to claim 11, **characterized in that** each tube (PT) of each even row (P) is placed and introduced between two corresponding tubes (DT) of each odd rows (D) and is offset with respect to the latter by at least half-diameter.

13. Unit, according to claim 1, **characterized in that** said operative head (31) is equipped with a central arm (50), which has N picking up elements (32) and with a lateral arm (51) with one picking up element (32).

14. Unit, according to claim 12, **characterized in that** said operative head (31) includes a first pusher (56) aimed at operating the central arm (50) to translate alternately in a direction (M), perpendicular to the direction (V) of the operative head (31) movement, and **in that** said operative head (31) includes also a second pusher (53) aimed at operating the lateral arm (51) to move between two separate operative configurations with respect to said central arm (50): first, union configuration (U1), in which the lateral arm (51) is contiguous to the central arm (50), and second, separated configuration (U2), in which the lateral arm (51) is moved far from the central arm (50).

15. Unit, according to claim 1 or 13 or 14, **characterized in that** said operative head (31) moves said central arm (50) and said lateral arm (51), the latter being in said first union configuration (U1), to said package (C) situated on said support seat (2), so as to pick up, in correspondence to their outer walls (TS), N+1 tubes (DT) from odd rows (D) by acting from the top through the window (F) of said wall (C2) of the package (C) by means of the relative N+1 picking up means (32) and **in that** said central arm (50) with said lateral arm (51), the latter being in said second separated configuration (U2) moved away by said second pusher (53), aimed at being positioned, by said first pusher (56), in correspondence to said N tubes (PT) of the even rows (P) to pick up from the top, N tubes (PT) from even rows (P) by acting from the top through the window (F) of said wall (C2) of the package (C) by means of the relative N picking up means (32).

16. Unit, according to claim 1, **characterized in that** it includes picking up means (32) formed by suction cups.

## Patentansprüche

1. Handhabungsvorrichtung zum Ergreifen von Tuben aus einer kastenähnlichen Schachtel und zu deren Ablage auf einem Förderband, wobei die besagte Vorrichtung (U) zwischen wenigstens einem Band für die Zuführung der besagten kastenähnlichen Schachteln (C) und wenigstens einem Förderband (B) angeordnet ist, wobei die besagten kastenähnlichen Schachteln (C) eine obere Abdeckung (C1) aufweisen, die für das Ergreifen der besagten Tuben (T) geöffnet ist, die in den Schachteln (C) derart angeordnet sind, daß ihre Öffnung (I) für das Einbringen des Produktes in Richtung auf die geöffnete obere Abdeckung (C1) ausgerichtet ist, **dadurch gekennzeichnet, daß** sie umfaßt: einen Stützsitz (2), der eine zugeordnete Schachtel (C) aus der Gruppe der besagten kastenähnlichen Schachteln, die von dem besagten zuführenden Förderband kommt, aufnimmt und stützt, wobei die besagte Schachtel (C) eine obere seitliche Wand (C2) aufweist, die mit einem Fenster (F) versehen ist, das der besagten geöffneten oberen Abdeckung (C1) benachbart angeordnet ist; eine Antriebseinheit (30), die in der Nähe des besagten Stützsitzes (2) angeordnet ist und die mit einem Handhabungskopf (31) ausgerüstet ist, der mit Greifmitteln (32) ausgestattet ist, wobei die besagte Antriebseinheit (30) dazu vorgesehen ist, den besagten Handhabungskopf (31) derart zu beaufschlagen, daß sich der letztere zu dem besagten Fenster (F) der besagten Schachtel (C), die sich in dem besagten Stützsitz (2) befindet, hin bewegt, um auf diese Weise die geöffnete obere Abdeckung (C1) in Richtung auf die Antriebseinheit (30) zu drehen, und die besagten Greifmittel (32) derart zu beaufschlagen, daß sie durch das besagte Fenster (F) hindurch die äußere seitliche Wand (TS) der besagten Tuben (T) von der Oberseite her ergreifen, wobei die besagte Antriebseinheit (30) auch dazu vorgesehen ist, daß sie den besagten Handhabungskopf (31) derart ansteuert, daß dieser in geeignetem Schritttakt zurückkehrt, um die besagten Tuben (T) aus der besagten Schachtel (C) durch die geöffnete obere Abdeckung (C1) hindurch zu entnehmen, und daß er zu dem besagten Förderband (B) zurückbewegt wird, um die besagten Tuben (T) auf diesem abzusetzen.

2. Vorrichtung gemäß Anspruch 1, **dadurch gekennzeichnet, daß** ein Raum (100), der auf einer Seite durch die besagte Antriebseinheit (30) und auf der anderen Seite durch den Stützsitz (2) an der Unterseite und durch das Förderband (B) an der Oberseite begrenzt wird, nur durch den besagten Handhabungskopf (31) eingenommen wird.

3. Vorrichtung gemäß Anspruch 2, **gekennzeichnet durch** Mittel, die geeignet sind, **durch** den besagten Raum (100) hindurch einen laminaren Luftstrom (200) vertikal abwärts zu senden, der sich **durch** das besagte Förderband (B) erstreckt, wobei der besagte Handhabungskopf (31) sich in unterschiedlichen operativen Konfigurationen zum Aufnehmen und zum Entnehmen von Tuben (T) aus der Schachtel (C) und zu deren Ablage auf dem Förderband (B) befindet, und **durch** die besagte Schachtel (C), die sich auf dem besagten Stützsitz (2) befindet.

4. Vorrichtung gemäß Anspruch 1, **dadurch gekennzeichnet, daß** die vordere sich bewegende Oberfläche (Z) des besagten Zuführ-Förderbandes eine Ebene (β) bildet, die in Bezug auf eine horizontale Ebene (y-y) um einen Winkel (α) geneigt ist, wobei die Öffnung des besagten Winkels (α) in Richtung auf die Antriebseinheit (30) gedreht ist, und **dadurch**, daß der besagte Stützsitz (2) eine Stützfläche (21) umfaßt, die zwischen zwei unterschiedlichen operativen Konfigurationen gedreht werden kann: einer ersten Ruhekonfiguration (O1), in der die besagte Stützfläche (21) senkrecht zur besagten Fläche (β) liegt, und einer zweiten operativen Konfiguration (02), in der die besagte Stützfläche (21) mit der besagten Fläche (β) ausgerichtet ist, um ein Schachtel (C) zu stützen.

5. Vorrichtung gemäß Anspruch 4, **dadurch gekennzeichnet, daß** der Wert des besagten Winkels (α) derart gewählt ist, daß die Tuben (T), die in der besagten Schachtel (C) enthalten sind, während der Vorwärtsbewegung der Schachtel (C) auf dem besagten Zuführ-Förderband und deren Transfer zum besagten Stützsitz (2) in einer horizontalen oder im wesentlichen horizontalen Richtung ausgerichtet sind.

6. Vorrichtung gemäß Anspruch 4, **dadurch gekennzeichnet, daß** die besagte Stützfläche (21) in der besagten ersten Ruhekonfiguration (O1) eine Auslaßöffnung (24) freigibt, um die von den Tuben (T) geleerten Schachteln (C) in Richtung einer Sammeleinheit zu entladen.

7. Vorrichtung gemäß den Ansprüchen 1 und 4, **dadurch gekennzeichnet, daß** sie Stütz- und Antriebsmittel (3) der besagten Antriebseinheit (30) umfaßt, die die letztere vom besagten Stützsitz (2) zum besagten Zuführ-Förderband (B) und umgekehrt in einer Richtung (S) bewegt, die senkrecht zur besagten Ebene (β) liegt.

8. Vorrichtung gemäß den Ansprüchen 1 und 4, **dadurch gekennzeichnet, daß** die besagte Antriebseinheit (30) den besagten Handhabungskopf (31) derart beaufschlagt, daß er eine Hin- und Herbewegung in einer Richtung (V) ausführt, die zur besagten Ebene (β) parallel liegt.

9. Vorrichtung gemäß Anspruch 1, **dadurch gekennzeichnet, daß** sie Steuermittel (8) umfaßt, die in der besagten Antriebseinheit (30) angeordnet sind und die den Handhabungskopf (31) derart anzusteuern, daß er sich während der Positionierung des besagten Handhabungskopfes (31) in Ausrichtung zum besagten Zuführ-Förderband (B) in Richtungen (W) dreht, wobei die Steuermittel (8) derart ausgebildet sind, daß sie die Tuben (T), die durch den besagten Handhabungskopf (31) aufgenommen werden, parallel dazu ausrichtet.

10. Vorrichtung gemäß Anspruch 9, **dadurch gekennzeichnet, daß** die besagten Rotations-Steuermittel (8) einen Stab (45) umfassen, der an seinem Ende einen Stift (47) trägt, der in einen Schlitz (48) eingeführt wird, der im Gehäuse (49) des Handhabungskopfes (31) vorgesehen ist, wobei der besagte Stab (45) durch einen Stellantrieb derart beaufschlagt wird, daß er durch die zugeordnete Bewegung besagten Stiftes (47) innerhalb des besagten Schlitzes (48) den Handhabungskopf (31) derart beaufschlagt, daß sich dieser um eine Drehachse (R) dreht.

11. Vorrichtung gemäß Anspruch 1, **dadurch gekennzeichnet, daß** die besagten Tuben (T) in den besagten Schachteln (C) derart angeordnet sind, daß sie mit der Öffnung (I) in Richtung auf die geöffnete obere Abdeckung (C1) gedreht sind und entlang aufeinanderfolgender ungerader Reihen (D) und gerader Reihen (P) angeordnet sind, wobei die besagten ungeraden Reihen (D) und geraden Reihen (P) übereinander angeordnet sind, beginnend von der unterer seitlichen Wand (C3) bis zur oberen seitlichen Fensterwand (C2), wobei jede der ungeraden Reihen (D) N+1 Tuben (DT) der besagten Tuben (T) enthält, die eine neben der anderen liegend angeordnet sind und wobei jede der geraden Reihen (P) N Tuben (PT) der besagten Tuben (T) enthält, die eine neben der anderen liegend angeordnet sind.

12. Vorrichtung gemäß Anspruch 11, **dadurch gekennzeichnet, daß** jede Tube (PT) einer geraden Reihen (P) zwischen je zwei entsprechende Tuben (DT) der ungeraden Reihen (D) plaziert ist und in Bezug auf die letzteren um wenigstens den halben Durchmesser versetzt angeordnet ist.

13. Vorrichtung gemäß Anspruch 1, **dadurch gekennzeichnet, daß** der besagte Handhabungskopf (31) mit einem zentralen Arm (50) ausgerüstet ist, der N Greifelemente (32) aufweist, und mit einem seitlichen Arm (51), der ein Greifelement (32) aufweist.

14. Vorrichtung gemäß Anspruch 12, **dadurch gekennzeichnet, daß** der besagte Handhabungskopf (31) einen ersten Schieber (56) umfaßt, der derart ausgebildet ist, daß er den zentralen Arm (50) beaufschlagt, so daß dieser sich in einer Richtung (M) hin und her bewegt, die zur Richtung (V) der Bewegung des Handhabungskopfes (31) senkrecht liegt, und **dadurch**, daß der besagte Handhabungskopf (31) außerdem einen zweiten Schieber (53) umfaßt, der derart ausgebildet ist, daß er den seitlichen Arm (51) beaufschlagt, so daß dieser sich zwischen zwei verschiedenen operativen Konfigurationen in Bezug auf den besagten zentralen Arm (50) hin und her bewegt: einer ersten Anschlußkonfiguration (U1), in der der seitliche Arm (51) am zentralen Arm (50) anliegt, und einer zweiten getrennten Konfiguration (U2), in der sich der seitliche Arm (51) weit vom zentralen Arm (50) entfernt befindet.

15. Vorrichtung gemäß Anspruch 1 oder 13 oder 14, **dadurch gekennzeichnet, daß** der besagte Handhabungskopf (31) den besagten zentralen Arm (50) und den besagten seitlichen Arm (51), wobei sich der letztere in der besagten ersten Anschlußkonfiguration (U1) befindet, zu der besagten Schachtel (C) bewegt, die auf dem besagten Stützsitz (2) angeordnet ist, um in Ausrichtung mit den äußeren Wänden (TS) N+1 Tuben (DT) aus den ungeraden Reihen (D) zu entnehmen, indem er von der Oberseite durch das Fenster (F) der besagten Wand (C2) der Schachtel (C) hindurch mittels der zugeordneten N+1 Greifelemente (32) agiert, und daß der besagte zentrale Arm (50) mit dem besagten seitlichen Arm (51), wobei der letztere sich in der besagten zweiten getrennten Konfiguration (U2) befindet, durch den besagten zweiten Schieber (53) weggerückt wird, so daß dieser durch den besagten ersten Schieber (56) in eine Position gebracht wird, in der er zu den besagten N Tuben (PT) der geraden Reihen (P) ausgerichtet ist, um von oben her N Tuben (PT) aus den geraden Reihen (P) zu entnehmen, indem er von der Oberseite durch das Fenster (F) der besagten Wand (C2) der Schachtel (C) hindurch mittels der zugeordneten N Greifelemente (32) agiert.

16. Vorrichtung gemäß Anspruch 1, **dadurch gekennzeichnet, daß** sie Greifelemente (32) umfaßt, die aus Saugschalen bestehen.

## Revendications

1. Unité de manipulation pour prélever des tubes dans un emballage de type boite et les placer sur une bande transporteuse, ladite unité de manipulation (U) étant située entre au moins une chaîne d'acheminement desdits emballages de type boite (C) et au moins une chaîne de transport (B), lesdits emballages de type boite (C) ayant une paroi supérieure (C1) ouverte pour prélever lesdits tubes (T), qui sont disposés dans lesdits emballages (C) avec l'ouverture (I) pour introduire le produit tournée vers la paroi supérieure ouverte (C1), **caractérisée en ce qu'**elle comprend un siège de support (2) qui reçoit et stabilise un emballage individuel (C) desdits emballages de type boîte, provenant de ladite chaîne d'acheminement, ledit emballage (C) ayant la paroi latérale supérieure (C2) munie d'une fenêtre (F) adjacente à ladite paroi supérieure ouverte (C1); une unité d'entraînement (30), située à proximité dudit siège de support (2) et muni d'une tête de manipulation (31) avec un moyen de prélèvement (32), ladite unité d'entraînement (30) étant destinée à actionner ladite tête de manipulation (31) pour transporter cette dernière jusqu'à ladite fenêtre (F) dudit emballage (C) située dans ledit siège de support (2), de façon à faire tourner la paroi supérieure ouverte (C1) vers l'unité d'entraînement (30) et actionner ledit moyen de prélèvement (32) pour saisir la paroi latérale externe (TS) desdits tubes (T) par la partie supérieure, à travers ladite fenêtre (F), ladite unité d'entraînement (30) étant également destinée à faire revenir la tête de manipulation (31), pas à pas, pour retirer lesdits tubes (T) dudit emballage (C) à travers ladite paroi supérieure ouverte (C1), et les amener à ladite chaîne de transport (B) pour y placer lesdits tubes (T).

2. Unité selon la revendication 1, **caractérisée en ce qu'**un espace (100), qui est délimité, d'un côté, par ladite unité d'entraînement (30) et, de l'autre côté, par le siège de support (2) dans la partie inférieure et par la chaîne de transport (B) dans la partie supérieure, n'est occupé que par ladite tête de manipulation (31).

3. Unité selon la revendication 2, **caractérisée par** un moyen pour envoyer et transporter, verticalement vers le bas à travers ledit espace (100), un flux d'air laminaire (200), qui s'étend à travers ladite chaîne de transport (B), ladite tête de manipulation (31) adoptant différentes configurations opérationnelles pour le prélèvement et le retrait de tubes (T) de l'emballage (C) et leur placement sur la chaîne de transport (B), et à travers ledit emballage (C) situé sur ledit siège de support (2).

4. Unité selon la revendication 1, **caractérisée en ce que** la surface (Z) se déplaçant en avant de ladite chaîne d'acheminement forme un plan (β) incliné par rapport à un plan horizontal (y-y) d'un angle (α), l'ouverture dudit angle (α) étant tournée vers l'unité d'entraînement (30), et **en ce que** ledit siège de support (29) comprend un plan de support (21), que l'on peut faire tourner entre deux configurations opérationnelles différentes : une première configuration de repos (01), dans laquelle ledit plan de support (21) est perpendiculaire audit plan (β), et une seconde configuration opérationnelle (02), dans laquelle ledit plan de support (21) est aligné avec ledit plan (β) pour supporter un emballage (C).

5. Unité selon la revendication 4, **caractérisée en ce que** la valeur dudit angle (α) est telle que les tubes (T) contenus dans ledit emballage (C) soit orientés dans une direction horizontale ou sensiblement horizontale au cours du mouvement en avant de l'emballage (C) sur ladite chaîne d'acheminement et du transfert vers ledit siège de support (2).

6. Unité selon la revendication 4, **caractérisée en ce que** ledit plan de support (21), dans ladite première configuration de repos (01), découvre une ouverture de sortie (24) afin de décharger les emballages (C) vidés de leurs tubes (T) vers une unité collectrice.

7. Unité selon les revendications 1 et 4, **caractérisée en ce qu'**elle comprend des moyens de support et de fonctionnement (3) de ladite unité d'entraînement (30) qui déplacent cette dernière dans un direction (S), perpendiculaire audit plan (β), dudit siège de support (2) à ladite chaîne de transport (B) et vice-versa.

8. Unité selon les revendications 1 et 4, **caractérisée en ce que** ladite unité d'entraînement (30) actionne ladite tête de manipulation (31) pour alterner le mouvement dans une direction (V) parallèle audit plan (β).

9. Unité selon la revendication 1, **caractérisée en ce qu'**elle comprend, situé dans l'unité d'entraînement (30), un moyen de commande (8) qui actionne la tête de manipulation (31) pour qu'elle tourne dans des sens (W), pendant le positionnement de ladite tête de manipulation (31) en correspondance avec ladite chaîne de transport (B), ledit moyen de commande (8) étant destiné à orienter les tubes (T), portés par ladite tête de manipulation (31), parallèlement à celle-ci.

10. Unité selon la revendication 9, **caractérisée en ce que** ledit moyen de commande de rotation (8) comprend une barre (45), qui présente, à son extrémité, une broche (47) introduite dans une fente (48) ménagée dans le corps (49) de la tête de manipulation (31), ladite barre (45) étant poussée par un actionneur pour actionner par le mouvement relatif de ladite broche (47) dans ladite fente (48), la tête de manipulation (31) pour qu'elle tourne sur un axe de rotation (R).

11. Unité selon la revendication 1, **caractérisée en ce que** lesdits tubes (T) sont arrangés dans lesdits emballages (C) avec l'ouverture (I) tournée vers la paroi supérieure ouverte (C1) et le long de rangées impaires (D) et de rangées paires (P) successives, lesdites rangées impaires (D) et lesdites rangées paires (P) étant situées l'une sur l'autre en commençant de la paroi latérale inférieure (C3) jusqu'à la paroi de fenêtre latérale supérieure (C2), chacune des rangées impaires (D) comprenant N+1 tubes (DT) desdits tubes (T), disposés l'un à côté de l'autre, et chacune des rangées paires (P) notamment N tubes (PT) desdits tubes (T) disposés l'un à côté de l'autre.

12. Unité selon la revendication 11, **caractérisée en ce que** chaque tube (PT) de chaque rangée paire (P) est placé et introduit entre deux tubes correspondants (DT) de chaque rangée impaire (D) et est décalé par rapport à ce dernier d'au moins un demi-diamètre.

13. Unité selon la revendication 1, **caractérisée en ce que** ladite tête de manipulation (31) est munie d'un bras central (50), qui a N éléments préleveurs (32), et d'un bras latéral (51) avec un élément préleveur (32).

14. Unité selon la revendication 12, **caractérisée en ce que** ladite tête de manipulation (31) comprend un premier poussoir (56) destiné à actionner ledit bras central (50) pour qu'il effectue une translation alternativement dans une direction (M), perpendiculaire à la direction (V) du mouvement de la tête de manipulation (31) et **en ce que** ladite tête de manipulation (31) comprend également un deuxième poussoir (53) destiné à actionner le bras latéral (51) pour qu'il se déplace entre deux configurations opérationnelles séparées par rapport audit bras central (50) : premièrement une configuration d'union (U1), dans laquelle le bras latéral (51) est contigu avec le bras central (50) et deuxièmement une configuration séparée (U2), dans laquelle le bras latéral (51) est déplacé à distance du bras central (50).

15. Unité selon la revendication 1 ou 13 ou 14, **caractérisée en ce que** ladite tête de manipulation (31) déplace ledit bras central (50) et ledit bras latéral (51), ce dernier étant, dans ladite première configuration d'union (U1) avec ledit emballage (C) situé sur ledit siège de support (2), de façon à prélever, en correspondance avec leurs parois externes (TS), N+1 tubes (DT) des rangées impaires (D) en agissant de la partie supérieure à travers la fenêtre (F) de ladite paroi (C2) de l'emballage (C) à l'aide des N+1 moyens de préleveurs connexes (32) et **en ce que** ledit bras central (50) avec ledit bras latéral (51), ce dernier étant dans ladite seconde configuration séparée (U2) écarté par ledit second poussoir (53), destiné à être positionné, par ledit premier poussoir (56), en correspondance avec lesdits N tubes (PT) des rangées paires (P) pour prélever de la partie supérieure, N tubes (PT) de rangées paires (P) en agissant de la partie supérieure à travers la fenêtre (F) de ladite paroi (C2) de l'emballage (C) à l'aide des N moyens de préleveurs connexes (32).

16. Unité selon la revendication 1, **caractérisée en ce qu'**elle comprend des moyens préleveurs (32) formés par des ventouses.
